# EUROPEAN PATENT SPECIFICATION

(11) **EP 2 029 040 B1**
(45) Date of publication and mention of the grant of the patent: **27.01.2016**
(21) Application number: 07795999.7
(22) Date of filing: 12.06.2007
(51) Int. Cl.: A61B 18/14

(54) **WIRE GUIDE SPHINCTEROTOME**
SPHINKTEROTOM MIT DRAHTFÜHRUNG
SPHINCTÉROTOME À FIL GUIDE

(30) Priority: 16.06.2006 US 814434 P
(43) Date of publication of application: 04.03.2009
(73) Proprietor: Cook Medical Technologies LLC, Bloomington, IN 47404 (US)
(72) Inventor: BLIND, Per-Jonas, 222 41 Lund (SE); SKERVEN, Gregory, J., Kernersville, NC 27284 (US)
(74) Representative: Garratt, Peter Douglas
(86) International application number: PCT/US2007/013746
(87) International publication number: WO 2007/149263

(56) References cited:
- WO-A-01/28445
- WO-A-01/54602
- WO-A-02/45609
- WO-A-98/27877
- WO-A-99/22658
- WO-A-2006/049970
- US-A1- 2003 208 219
- US-B1- 6 712 817

## Description

### FIELD OF THE INVENTION

The present invention relates to medical devices, and more particularly to medical devices, such as sphincterotomes and wire guides, used in surgical procedures.

### BACKGROUND

Medical devices, such as catheters delivered over wire guides, are used in the pancreatobiliary system for endoscopic or other minimally invasive surgery.

Such devices are described for example in documents US 20030032936 or WO 9922658.

Typically, an Endoscopic Retrograde Cholangiopancreatography (ERCP) procedure is performed when the Sphincter of Oddi becomes constricted due to disease or trauma by introducing a catheter device from a duodenoscope through the ampullary orifice (Papilla of Vater) and into the biliary tree, which includes the bile duct, pancreatic duct and hepatic ducts of the liver. In the ERCP procedure, a wire guide is first introduced into the biliary tree and the cannulation device, which is usually a sphincterotome/papllitome or ECRP catheter, is introduced over the wire guide and into the biliary tree to perform a first operation, which could be diagnostic in nature, such as injecting contrast media, or for therapeutic purposes, such as enlarging the ampullary orifice. Introducing the wire guide prior to or at the same time as the sphincterotome allows for cannulation of closed sphincters and other strictures due to the smaller diameter of the wire guide as compared to the sphincterotome.

The sphincterotome may be used both to cannulate the ductal system and to enlarge the opening by delivery of electrical current to a bowed cutting wire at the distal end of the sphincterotome. Exemplary sphincterotomes typically include at least two lumens extending through the shaft thereof. One lumen is provided for an operating wire that is connected to the cutting wire and the other lumen is provided for the wire guide. Accordingly, typical sphincterotomes have a relatively large cross-section - much larger than a typical wire guide. The wire guide is usually left in place while the sphincterotome is advanced, operated and then removed. The wire guide can be used to provide subsequent access for other devices when additional medical procedures are performed, such as to remove a stone, to open a stricture, or to sample tissue.

Complications are associated with the ERCP procedure and accessing the papilla of the pancreatic duct with the known medical devices described above, particularly multi-lumen sphincterotomes having a relatively large cross-section. Complications include acute pancreatitis, bleeding, duodenal perforation, cholangitis, etc. generally due to mechanical and thermal trauma of the papilla. Although complication rates are generally low, decreasing trauma to the papilla will help further lower the complication rates. The present invention provides a wire guide adapted to cannulate a stricture without requiring a separate device, such as a sphincterotome having a cutting wire, to perform the procedure. The present invention also provides a single lumen catheter suitable for use with the wire guide of the present invention.

### BRIEF SUMMARY

The scope of the present invention is set forth in the appended claims. A medical system configured for cannulation of a lumen having a stricture is provided. The medical system includes an elongate sheath having a proximal portion, a distal portion and a first lumen at least partially extending through the sheath. The medical system also includes a wire guide having a cutting portion, a non-cutting tip portion and a non-cutting shaft portion proximal to the cutting portion. The wire guide extends at least partially through the first lumen and at least a portion of the cutting portion is positionable outside the sheath to cannulate the stricture.

### BRIEF DESCRIPTION OF THE DRAWINGS

Embodiments of the present invention will now be described by way of example with reference to the accompanying drawings, in which:
FIG. IA is a side view of a catheter handle for a wire guide;
FIG. IB is an enlarged side view of a distal portion of a distal end of a catheter with a wire guide extending through a lumen thereof;
FIG. 2 is a partial side view of the wire guide shown in FIG. 1B;
FIG. 3 is a partial side view of an alternative embodiment of the wire guide and catheter;

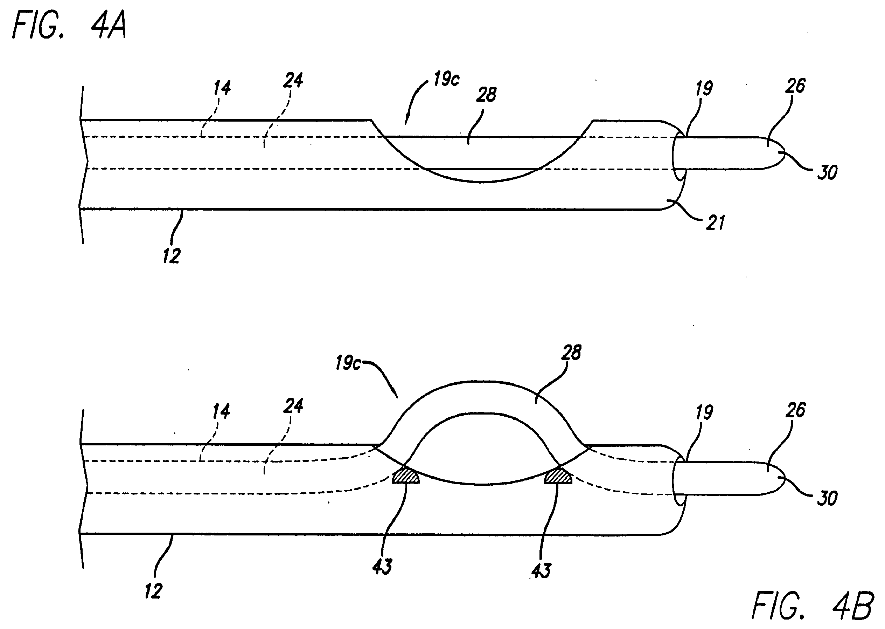

FIG. 4A is a partial side view of an alternative embodiment of the wire guide and catheter;
FIG. 4B is a partial side view of an alternative embodiment of the wire guide and catheter;
FIG. 5A is a partial side view of the cutting portion of the wire guide;
FIG. 5B is a partial side view of an alternative embodiment of the cutting portion of the wire guide;
FIG. 5C is a cross- sectional view of the cutting portion of the wire guide shown in FIG. 5B through A-A;

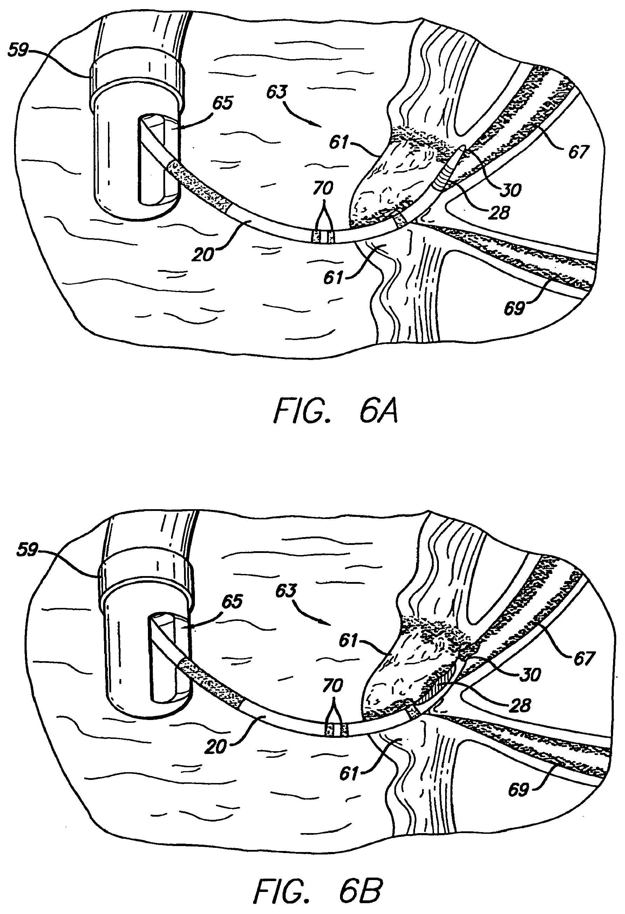

FIG. 6A is a partial view of the wire guide shown in FIG. 2 being used to cannulate a stricture in the biliary system; and
FIG. 6B is a partial view of the wire guide shown in FIG. 4A being used to cannulate a stricture in the biliary system.

### DETAILED DESCRIPTION OF THE INVENTION

The invention is described with reference to the drawings in which like elements are referred to by like numerals. The relationship and functioning of the various elements of this invention are better understood by the following detailed description. However, the embodiments of this invention as described below are by way of example only, and the invention is not limited to the embodiments illustrated in the drawings. It should also be understood that the drawings are not to scale and in certain instances details have been omitted, which are not necessary for an understanding of the present invention, such as conventional details of fabrication and assembly.

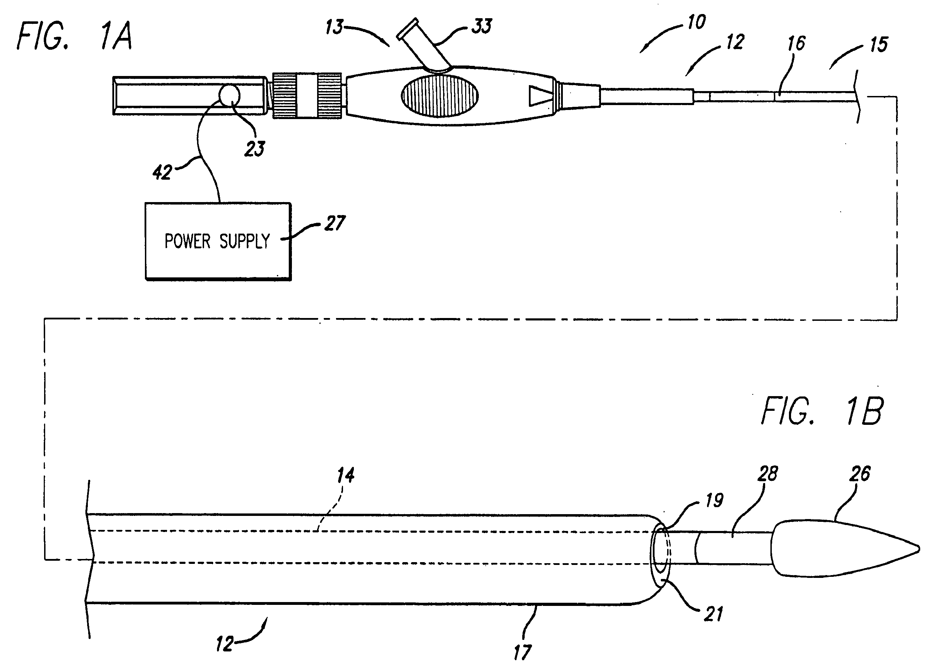

FIGS. 1A and 1B illustrate a medical system 10 according to the present invention. The system 10 includes a catheter 12 having a handle 13 and a wire guide 20 disposed through a lumen 14 of the catheter 12. The catheter 12 includes an elongate sheath 15 having a proximal portion 16 and a distal portion 17. The lumen 14 includes one or more ports exiting the lumen 14. As shown in FIG. 1B, the lumen 14 has a port 19 opening at a distal end 21 of the catheter 12.

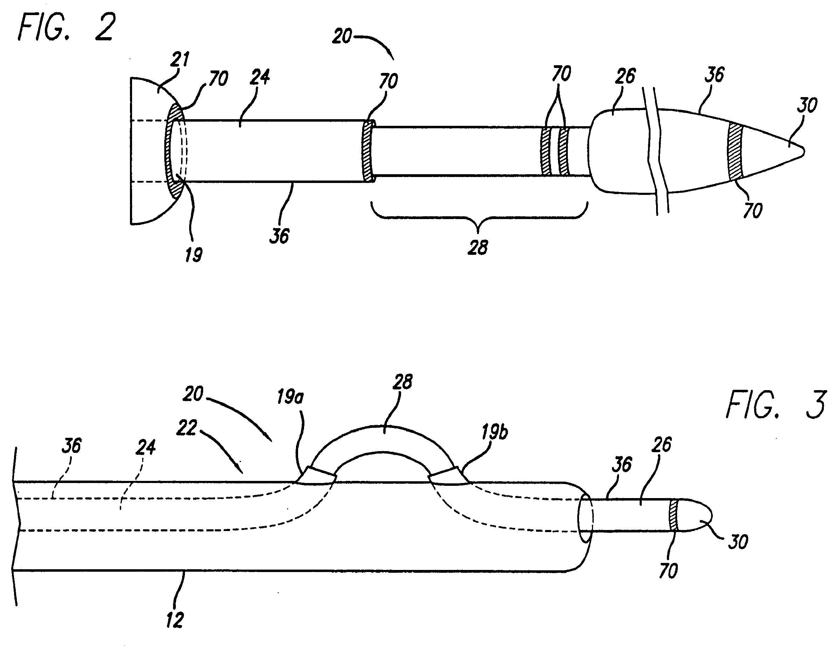

FIGS. 2 and 3 illustrate embodiments of the wire guide 20. The wire guide 20 provides both navigation through body passageways and cannulation once the wire guide 20 is properly positioned within the passageway. A separate cutting wire for cannulation is not necessary. The wire guide 20 includes an elongate shaft 22 having a non-cutting proximal portion 24 and a distal portion 26 for navigation through body passageways. The wire guide 20 also includes a cutting portion 28. A connector 23 may be removably connected to the proximal portion 24 of the wire guide 20 (see FIG. 1A). The connector 23 connects a power supply 27 to the wire guide 20 (described below). The distal portion 26 of the wire guide 20 includes a non-cutting tip 30 having an atraumatic profile. For example, the tip 30 may be tapered, curvilinear, rounded and the like. This configuration provides for smooth entry into body passageways having strictures or blockages or tortuous configurations. The atraumatic tip profile also helps to prevent the distal tip 30 from inadvertently snagging and thereby unnecessarily traumatizing surrounding body tissues. The wire guide 20 may also include a contrast dye injection port 33 as shown in FIG. 1A. The injection port 33 may be connected to a separate lumen or connected to the wire guide lumen 14.

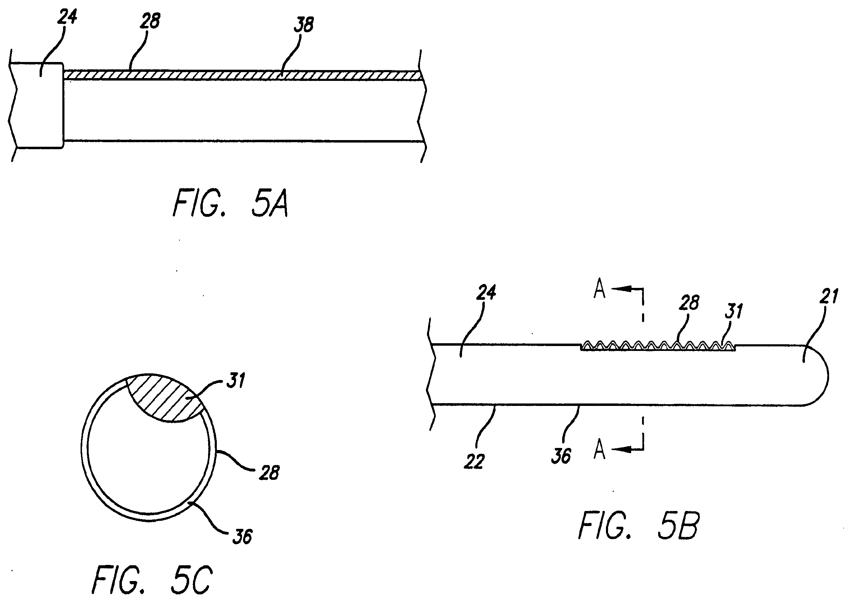

The wire guide 20, except for the features as described herein and shown in the figures, may be any type of wire guide known to one of skill in the art. By way of non-limiting example, the non-cutting portion of wire guide 20 may be a helical wire, a simple wire, a braided wire, and the like. In some embodiments, the cutting portion 28 of the wire guide 20 may be a simple wire having a smooth profile. Alternatively, the cutting portion 28 of the wire guide 20 may have a patterned profile to maximize the current delivery to portions of the tissue. The cutting portion 28 of the wire guide 20 may include a region 38 of highly electroconductive material extending longitudinally along the cutting portion 28, as shown in FIG. 5A. The region 38 may be used to concentrate the current delivery to a specific site at the stricture rather than having current radiating in all directions from the cutting portion 28. Alternatively, the cutting portion 28 of the wire guide 20 may be a region 31 extending longitudinally along the shaft 22 of the wire guide 20 where only the region 31 may be used for cannulation. For example, the region 31 may extend longitudinally along a portion of the shaft 22 but not around the entire circumference of the shaft 22 (see FIG. 5B and 5C). In this embodiment, the cutting portion 28 of the wire guide 20 may be directionally targeted to the tissue to be cannulated. In some embodiments, the proximal portion 24 and/or the cutting portion 28 of the elongate shaft 22 may be relatively stiff or have a higher durometer to provide greater control.

At least a portion of the wire guide 20 is disposed in the lumen 14 of the catheter 12. The wire guide 20 may be provided for use in a long wire or short wire exchange procedure. In the short wire exchange system, the distal portion 26 of the wire guide 20 is disposed in the lumen 14 and the proximal portion of the wire guide 20 is positioned external to the catheter 12. In the short wire exchange system, the wire guide 20 may be remotely disconnected from the catheter 12 at the work site, such as within a lumen or other bodily passage/cavity, and additional devices may be placed over the wire guide 20. A short wire exchange system is described in U.S. Publication No. 2005/0070794. The wire guide 20 may also be disposed in the lumen 14 of the catheter 12 in a conventional long wire exchange as will be understood by one skilled in the art. Any type of catheter configured for use with a wire guide may be used with the wire guide of the present invention.

As shown in FIG. 2, the distal portion 26 of the wire guide 20 extends distally from the port 19 at the distal end 21 of the catheter 12. The wire guide 20 may be extended distally so that the cutting portion 28 is disposed distal of the port 19 and can be used to cannulate tissue as described below. In some embodiments, the tip 30 may be tapered and extend distally beyond the cutting portion 28 between about 2.5 to 25 centimeters (cm), preferably about 5 to 10 cm. The cutting portion 28 may be about 0.5 to 5 cm in length.

As shown in FIG. 3, the cutting portion 28 of the wire guide 20 extents externally from the lumen 14 of the catheter 12 through side ports 19a and 19b so that the cutting portion 28 extends generally axially with respect to the catheter 12. At least a portion of the distal portion 26 of the wire guide 20 may also extend distally from the port 19 of the catheter 12. The port 19b may be located about 5-20 cm proximal from the distal end 21 of the catheter 12. The spacing between the ports 19a and 19b may be the same length as the cutting portion 28, or may be longer or shorter than the cutting portion 28. For example, the cutting portion 28 may be about 0.5 to 5 cm in length. In some embodiments, the port 19a may be spaced apart from the port 19b about 0.5 to about 3 cm. The lengths provided above are meant to be non-limiting examples and one skilled in the art will understand that other lengths for the cutting portion 28, the tip 30 and the distance between the ports are possible. The wire guide 20 is configured to be movably disposed in the catheter 12. The wire guide 20 may be extended distally so that the cutting portion 28 is exposed external to the catheter 12 and the distal portion 26 extends distally through the port 19. After cannulating the sphincter, the wire guide 20 may be retracted proximally so that the distal portion 26 of the wire guide 20 may be retracted into the catheter 12 so that the wire guide 20 is also removed proximally through the ports 19b and 19a. If desired, the wire guide 20 may be moved distally through the lumen 14 and extended through the port 19, bypassing the ports 19a and 19b upon re-extension to be used as a wire guide or for cannulating an additional stricture. For subsequent cannulation, the cutting portion 28 of the wire guide 20 may be extended through the port 19 and be energized.

In some embodiments, the cutting portion 28 of the wire guide 20 may be movably disposed in the catheter 12 and configured to extend externally from the lumen 14 of the catheter 12 through a channel 19c so that the cutting portion 28 extends generally axially with respect to the catheter 12 as shown in FIGS. 5A and 5B. Similar to the embodiments described above, the cutting portion 28 of the wire guide 20 extends external to the catheter 12 so that a stricture may be cannulated. In some embodiments having a channel 19c, the cutting portion 28 may be bowed to extend beyond the catheter 12. The cutting portion 28 may be bowed by using ramp-like structures 43 on either side of the channel 19c to angle the cutting portion 28 of the wire guide 20 as the cutting portion exits and reenters the lumen 14. The distal portion 26 may extend distally through the port 19 similar to the embodiments described above. The channel 19c may be located about 5-20 cm proximal from the distal end 21 of the catheter 12. The length of the cutting portion 28 exposed in the channel 19 may be about 0.5 to about 5 cm. Similar to the embodiment having a pair of ports 19a and 19b shown in FIG. 3, the wire guide 20 may be retracted proximally through the channel 19c so that the tip 30 of the wire guide 20 is completely withdrawn into the lumen 14. The wire guide 20 may be re-extended through the channel 19c and in the embodiments having the ramp-like structure 43, the wire guide 20 may be redirected at an angle along the structure 43 and away from the distal end 21 of the catheter 12.

The wire guide 20 may have conventional dimensions and be made from conventional materials where at least some of the components are capable of conducting current. For example, the length of the wire guide 20 may range from about 40 to 480 cm. An outside diameter of the wire guide 20 may range from about 0.020 to 0.13 cm (0.008 to 0.05 inches).

Examples of suitable materials for forming the portions of the wire guide 20 that are electroconductive include, but are not limited to stainless steel, tantalum, nitinol; gold, silver, tungsten, platinum, inconel, cobalt-chromium alloys and iridium, all of which are commercially available metals or alloys used in the fabrication of medical devices. Other portions of the wire guide may be formed from a medically-acceptable polymer. For example, exemplary polymers include, but are not limed to, cellulose acetate, cellulose nitrate, silicone, polyethylene, high density polyethylene, polyethylene teraphthalate, polyurethane, polytetrafluoroethylene, polyamide, polyester, polyorthoester, polyvinyl chloride (PVC), polypropylene, acrylonitrile-butadienestyrene (ABS), polycarbonate, polyurethane, nylon silicone, and polyanhydride. The wire guide 20 may be manufactured using conventional techniques. Examples of wire guides having features that may be included in the present invention include the FUSION^{®} System, DASH^{®} Direct Access System, OMNI devices, MINI-TOME PC^{®} and the like (available from Cook Endoscopy, Winston-Salem, N.C.)

The wire guide 20 or portions thereof may also be coated to provide for smoother entry into a stricture and to provide an insulating layer 36 on the wire guide 20. In some embodiments, the insulating layer 36 may cover the entire wire guide 20 with the exception of the cutting portion 28, which remains bare to convey current to the tissue (described below). The insulating layer 36 may be made from any insulating material suitable to protect against electrical hazards and may be selected based on the degree of insulation required and according to Standards IEC 60601-2-2, IEC 601-2-18 and AAMI HF 18. In some embodiments the insulating layer may comprise a polymer, for example, polytetrafluoroethlyene, polyimide, fluoropolymer, or PEBAX. A plurality of insulating layers 36 may also be included. The plurality of layers 36 may be made from the same or different materials. The plurality of layers 36 may cover a portion of the wire guide 20 and the thickness and number of layers at different portions of the wire guide 20 may vary. For example, the tip 30 may include more insulating layers 36 than the proximal portion 24 of the wire guide 20 in embodiments where the proximal portion 24 is generally disposed within catheter 12 and is insulated thereby. In some embodiments, the cutting portion 28 may extend only along the region 31 and the remaining circumferential area of the shaft 22 may include at least one layer 36.

Radiopaque materials may be added to the layer 36. Also, radiopaque materials may be placed directly on or within portions of the wire guide 20 and the catheter 12. For example, radiopaque materials may be placed near both ends of the cutting portion 28 and/or along the cutting portion 28 so that the position of the cutting portion 28 may be viewable by the wire guide operator using fluoroscopy. In the embodiment shown in FIG. 2, one or more bands 70 are present on the wire guide 20 and the catheter 12. Radiopaque markers having different sizes and/or different shapes may be used to identify different portions of the wire guide 20 and for positioning the cutting portion 28, as will be understood by one skilled in the art.

Several examples of suitable radiopaque materials and markers are known in the art, and any suitable material and/or marker can be utilized in the present invention. Common radiopaque materials include barium sulfate, bismuth subcarbonate, and zirconium dioxide. Other radiopaque elements include: cadmium, tungsten, gold, tantalum, bismuth, platinum, iridium, and rhodium. In one embodiment, iodine may be employed for its radiopacity and antimicrobial properties. Radiopacity is typically determined by fluoroscope or x-ray film. Radiopaque, physiologically compatible materials include metals and alloys selected from the Platinum Group metals, especially platinum, rhodium, palladium, rhenium, as well as tungsten, gold, silver, tantalum, and alloys of these metals. These metals have significant radiopacity and in their alloys may be tailored to accomplish an appropriate blend of flexibility and stiffness. They are also largely biocompatible. For example, a platinum/tungsten alloy, e.g., 8% tungsten and the remainder platinum may be used.

As shown in FIG. 1A, the proximal portion 24 of the wire guide 20 may be removably connected to the power source 27 for providing current to the cutting portion 28 for cannulating the stricture. The wire guide 20 may be used as a conventional wire guide when no current is supplied to the wire guide and the wire guide 20 may be used for cannulation when current is supplied. The cutting portion 28 may be activated and deactivated by the power source 27 itself or by removably connecting the power source 27 through the connector 23 via a lead 42. The power source 27 may be any power source known to one of skill in the art. For example, the power supply 27 may be a conventional power supply having conventional control circuitry to provide a constant or modulated AC or DC signal. The power may also be supplied as an RF signal. The signal is transmitted by the lead 42 to the connector 23 and to the wire guide 20. The connector 23 may be removable to permit devices, such as catheters, to be advanced over the proximal end of the wire guide 20. The connector 23 may also be rotatable to allow the wire guide 20 to be rotated and steered during navigation to the body location. Alternatively, the wire guide 20 may be positioned for cutting prior to connection to the power source 27. Exemplary power sources and connectors for use with a wire guide are described in U.S. Patent No. 6,602,250, which is incorporated by reference herein in its entirety.

An exemplary procedure utilizing the wire guide 20, for example in accessing the biliary system via the Sphincter of Oddi is shown in FIG. 5 and is described as follows. An endoscope 59 is advanced into the patient and positioned near the Sphincter of Oddi 61 in the Papilla of Vater 63. The endoscope 59 is positioned to allow viewing of sphincter 61 as is known. Next, the catheter 12 and the wire guide 20 are extended out of opening 65 in endoscope 59. The wire guide 20 is extended into engagement with sphincter 61 by inserting the tip 30 of the wire guide 20 into the Ampulla of Vater, which communicates with the common bile duct 67 and the pancreatic duct 69. The tip 30 may be extended into the Ampulla of Vater until the cutting portion 28 is aligned with the stricture to be cannulated. As used herein, the term stricture refers to any narrowing of a lumen in relation to an adjacent lumen portion. For example, the stricture may be a sphincter opening where the diameter of the sphincter opening is smaller than the adjacent lumen or the stricture may be an abnormal narrowing of a lumen due to a tumor and the like. The cutting portion 28 of the wire guide 20 may be oriented using the markers 70 to position the cutting portion 28, for example, in the center of sphincter 61 so that the cutting plane is oriented transversely with respect to the central Ampulla of Vater. Similarly, in embodiments having the cutting portion 28 of the wire guide 20 disposed in the channel 19c, the open portion of the channel having the cutting portion 28 exposed to the tissue may be aligned so that the cutting portion 28 within the channel 19c is positioned at the tissue for subsequent cannulation. The desired orientation will depend on the stricture and the individual anatomy of each patient. Once the cutting portion 28 is properly oriented, the power source 27 may be connected to the connector 23 and/or turned on to supply power so that the tissue may be cannulated with the cutting portion 28. Any tissue not in contact with the cutting portion 28 of the wire guide 20 will be protected by the insulating layer 36, the catheter 12 or both. After cannulating the stricture, the power source 27 may be turned off and in some embodiments, the connector 23 may be disconnected from the proximal portion 24 of the wire guide 20. Additional medical procedures may be conducted using the wire guide 20 in the non-energized state as a traditional wire guide.

Unless otherwise indicated, all ordinary words and terms used herein shall take their customary meaning as defined in The New Shorter Oxford English Dictionary, 1993 editi*on.* All technical terms shall take on their customary meaning as established by the appropriate technical discipline utilized by those normally skilled in that particular art area. All medical terms shall take their meaning as defined by Stedman's Medical Dictionary, 27th editi*on.*

It is therefore intended that the foregoing detailed description be regarded as illustrative rather than limiting, and that it be understood that it is the following claims, including all equivalents, that are intended to define the scope of this invention.

## Claims

1. A medical system configured for cannulation of a body passageway having a stricture, the system comprising:
an elongate sheath (15) comprising a proximal portion (16), a distal portion (17) and a lumen (14) at least partially extending through the sheath and connected to a port (19) in the distal portion of the elongate sheath; and
a wire guide (20) movable relative to the elongate sheath and distally extendable through the port, the wire guide extending at least partially through the lumen (14);
**characterised by** the wire guide comprising a cutting portion (28) and a non-cutting tip portion (30) distal to the cutting portion, the tip portion comprising an atraumatic profile and a non-cutting shaft portion (24) proximal to the cutting portion;
wherein the tip portion is distally extendable from a distal end of the sheath through the port (19) and configured to navigate the body passageway; and
wherein at least a portion of the cutting portion is extendable through a side port (19a, b, c) in the sheath and is positionable outside the sheath to cannulate the stricture.

2. The medical system of claim 1, wherein the cutting portion comprises an electroconductive material.

3. The medical system of claim 2, wherein the wire guide is operatively connected to a power source (27) for energizing the cutting portion applicable as a cutting element

4. The medical system of claim 2, wherein non-cutting shaft and tip portions of the wire guide each comprise an insulating layer.

5. The medical system of claim 4, wherein the insulating layer comprises polytetrafluoroethylene.

6. The medical system of claim 1, wherein the cutting portion includes an electroconductive region extending generally axially with respect to the sheath.

7. The medical system of claim 1, wherein the cutting portion is distally extendable through the port (19).

8. The medical system of claim 1, wherein the side port comprises a pair of side ports exiting the sheath.

9. The medical system of claim 1, wherein the side port comprises an elongated side port forming a channel in the sheath.

10. The medical system of claim 1, wherein the wire guide includes a tapered tip portion.

11. The medical system of claim 1, wherein the cutting portion is about 5 to 30 millimeters in length.

## Patentansprüche

1. Medizinisches System, das zur Kanülierung eines Körpergangs, der eine Striktur aufweist, ausgebildet ist, wobei das System Folgendes umfasst:
eine langgestreckte Hülle (15), umfassend einen proximalen Abschnitt (16), einen distalen Abschnitt (17) und ein Lumen (14), das sich zumindest teilweise durch die Hülle erstreckt und mit einer Anschlussöffnung (19) in dem distalen Abschnitt der langgestreckten Hülle verbunden ist; und
eine Drahtführung (20), die bezüglich der langgestreckten Hülle beweglich ist und distal durch die Anschlussöffnung vorgeschoben werden kann, wobei sich die Drahtführung zumindest teilweise durch das Lumen (14) erstreckt;
**dadurch gekennzeichnet, dass** die Drahtführung einen Schneidabschnitt (28) und einen nicht schneidenden Spitzenabschnitt (30) distal zu dem Schneidabschnitt umfasst, wobei der Spitzenabschnitt ein atraumatisches Profil und einen nicht schneidenden Schaftabschnitt (24) proximal zu dem Schneidabschnitt umfasst;
worin der Spitzenabschnitt von einem distalen Ende der Hülle distal durch die Anschlussöffnung (19) vorgeschoben werden kann und zur Navigation durch den Körpergang ausgebildet ist; und
worin zumindest ein Teil des Schneidabschnitts durch eine Seitenanschlussöffnung (19a, b, c) in der Hülle vorgeschoben werden kann und zur Kanülierung der Striktur außerhalb der Hülle positionierbar ist.

2. Medizinisches System nach Anspruch 1, worin der Schneidabschnitt ein elektrisch leitfähiges Material umfasst.

3. Medizinisches System nach Anspruch 2, worin die Drahtführung zur Erregung des Schneidabschnitts, der als Schneidelement anwendbar ist, mit einer Stromquelle (27) in Wirkverbindung steht.

4. Medizinisches System nach Anspruch 2, worin nicht schneidende Schaft- und Spitzenabschnitte der Drahtführung jeweils eine Isolierschicht umfassen.

5. Medizinisches System nach Anspruch 4, worin die Isolierschicht Polytetrafluorethylen umfasst.

6. Medizinisches System nach Anspruch 1, worin der Schneidabschnitt eine elektrisch leitfähige Region aufweist, die sich allgemein axial bezüglich der Hülle erstreckt.

7. Medizinisches System nach Anspruch 1, worin der Schneidabschnitt distal durch die Anschlussöffnung (19) vorschiebbar ist.

8. Medizinisches System nach Anspruch 1, worin die Seitenanschlussöffnung ein Paar von Seitenanschlussöffnungen umfasst, welche die Hülle verlassen.

9. Medizinisches System nach Anspruch 1, worin die Seitenanschlussöffnung eine langgestreckte Seitenanschlussöffnung umfasst, die einen Kanal in der Hülle formt.

10. Medizinisches System nach Anspruch 1, worin die Drahtführung einen verjüngten Spitzenabschnitt aufweist.

11. Medizinisches System nach Anspruch 1, worin der Schneidabschnitt eine Länge von ungefähr 5 bis 30 Millimeter aufweist.

## Revendications

1. Système médical configuré pour la pose d'une canule dans un passage corporel comportant une sténose, le système comprenant :
une gaine allongée (15) comprenant une partie proximale (16), une partie distale (17) et une lumière (14) s'étendant au moins partiellement à travers la gaine et raccordée à un orifice (19) dans la partie distale de la gaine allongée ; et
un fil-guide (20) déplaçable par rapport à la gaine allongée et extensible dans le sens distal à travers l'orifice, le fil-guide s'étendant au moins partiellement à travers la lumière (14) ;
**caractérisé en ce que** le fil-guide comprend une partie coupante (28) et une partie terminale non coupante (30) distale vis-à-vis de la partie coupante, la partie terminale comprenant un profil atraumatique et une partie tige non coupante (24) proximale vis-à-vis de la partie coupante ;
la partie terminale étant extensible dans le sens distal à partir d'une extrémité distale de la gaine à travers l'orifice (19) et configurée pour se déplacer dans le passage corporel ; et
au moins une partie de la partie coupante étant extensible à travers un orifice latéral (19a, b, c) dans la gaine et pouvant être positionnée à l'extérieur de la gaine afin de poser une canule au niveau de la sténose.

2. Système médical selon la revendication 1, dans lequel la partie coupante comprend un matériau électroconducteur.

3. Système médical selon la revendication 2, dans lequel le fil-guide est raccordé de manière fonctionnelle à une source d'alimentation (27) afin d'alimenter en énergie la partie coupante pouvant être employée en tant qu'élément coupant.

4. Système médical selon la revendication 2, dans lequel les parties tige et terminale non coupantes du fil-guide comprennent chacune une couche isolante.

5. Système médical selon la revendication 4, dans lequel la couche isolante comprend du polytétrafluoroéthylène.

6. Système médical selon la revendication 1, dans lequel la partie coupante comprend une région électroconductrice s'étendant généralement axialement par rapport à la gaine.

7. Système médical selon la revendication 1, dans lequel la partie coupante est extensible dans le sens distal à travers l'orifice (19).

8. Système médical selon la revendication 1, dans lequel l'orifice latéral comprend une paire d'orifices latéraux débouchant hors de la gaine.

9. Système médical selon la revendication 1, dans lequel l'orifice latéral comprend un orifice latéral allongé formant un canal dans la gaine.

10. Système médical selon la revendication 1, dans lequel le fil-guide comprend une partie terminale effilée.

11. Système médical selon la revendication 1, dans lequel la partie coupante présente une longueur d'environ 5 à 30 millimètres.
